# EUROPÄISCHE PATENTANMELDUNG

(11) **EP 0 967 474 A1**
(43) Veröffentlichungstag der Anmeldung: **29.12.1999**
(21) Anmeldenummer: 99112017.1
(22) Anmeldetag: 22.06.1999
(51) Int. Cl.: G01N 9/02, G01N 33/38

(54) **Verfahren und Vorrichtung zur Ermittlung der Dichte von Suspensionen**

(30) Priorität: 26.06.1998 DE 19828530
(71) Anmelder: Bilfinger + Berger Bauaktiengesellschaft, 68165 Mannheim (DE)
(72) Erfinder: Häffner, Willi, 68542 Heddesheim (DE); Lindemann, Wilhelm Dipl.-Ing, 68307 Mannheim (DE); Seidemann, Joachim, 06427 Kayna (DE); Uhlmann, Jürgen Dipl.-Ing, 07549 Gera (DE)
(74) Vertreter: Schmid, Rudolf, Dipl.-Ing., Patentanwalt

(57) **Zusammenfassung**

Vorgeschlagen wird ein Verfahren und eine Vorrichtung zur Ermittlung der Dichte feinkörniger, im Tiefbau verwendeter Suspensionen, wie insbesondere Bentonit-Wasser-Suspensionen, Bentonit-Zement-Wasser-Suspensionen, Bentonit-Wasser-Zement-Sand-Suspensionen und dergleichen. Erfindungsgemäß wird dazu die Suspensionsdichte in einem Suspensionsbehälter mit einem definierten Querschnitt durch Messung der Füllstandshöhe der Suspension und Wägung des Suspensionsgewichts ermittelt.

## Beschreibung

Die Erfindung betrifft ein Verfahren und eine Vorrichtung zur Ermittlung der Dichte feinkörniger, im Tiefbau verwendeter Suspensionen, wie insbesondere Bentonit-Wasser-Suspensionen, Bentonit-Zement-Wasser-Suspensionen, Bentonit-Wasser-Zement-Sand-Suspensionen und dergleichen.

Solche Suspensionen werden im Tiefbau in vielfältiger Weise verwendet. So benutzt man Bentonit-Wasser-Suspensionen aufgrund ihrer Stützwirkung unter anderem zum flüssigkeitsgestützten Bohren, z.B. zur Herstellung von Bohrpfählen, oder zur Herstellung von Lamellen von Schlitzwänden im Zwei-Phasen-Verfahren. Bentonit-Zement-Wasser-Suspensionen besitzen ebenfalls einen grossen Verwendungsbereich. Genannt seien hier beispielhaft die Herstellung von Schlitzwänden, Pfählen und Dichtwänden im Ein-Phasen-Verfahren und die Verwendung solcher Suspensionen beim Düsenstrahlverfahren.

Aus Gründen der Wirtschaftlichkeit ist man bemüht, gebrauchte Suspension nicht sofort zu entsorgen, da dies aufgrund der grossen Mengen sehr kostspielig ist, sondern die Suspension mit Hilfe einer Regenerieranlage so aufzubereiten, dass sie möglichst oft im Kreislauf verwendet werden kann. Während des Bohr- oder Schlitzvorgangs lädt sich die Suspension mit Bodenteilchen auf und verändert dadurch ihre physikalischen Eigenschaften. Die Regeneration einer solchen gebrauchten Suspension erfolgt in der Regel durch Abtrennung unerwünschter Bodenfraktionen z.B. durch Siebe und Zyklone. Die regenerierte Suspension wird dann mit frisch angemischter Suspension vermischt und wieder verwendet.

Der Erfolg von Bauverfahren, in denen feinkörnige Suspensionen verwendet werden, hängt nun entscheidend davon ab, wie genau die Einhaltung der vorgegebenen Eigenschaften der Suspension kontrolliert werden kann. Hier ist insbesondere die Kontrolle der Dichte der Suspension von Bedeutung. Die Dichte sowohl der frisch angemischten als auch der regenerierten Suspension muss daher während des Bauvorgangs ständig kontrolliert werden. Beispielhaft für die Wichtigkeit der Dichtekontrolle seien hier zwei Bauverfahren genannt, nämlich die Herstellung einer Schlitzwand und die Bohrpfahlherstellung.

Ein Herstellverfahren für Gründungspfähle ohne Verrohrung gewinnt gegenüber den klassischen Verfahren, bei denen die Bohrlochwandung des Pfahls durch ein Stahlrohr gestützt wird, zunehmend an Bedeutung. Dabei erfolgt der Bodenaushub unter Verwendung einer Stützflüssigkeit aus Wasser und Bentonit oder Zement und ähnlichen Stoffen. Nach Erreichen der Endtiefe wird die Bewehrung des Pfahls eingebaut und anschließend der Pfahlbeton im Kontraktorverfahren eingebracht. Dabei wird die Stützflüssigkeit durch den Pfahlbeton verdrängt. Um einen homogenen Pfahlbeton herstellen zu können, muss die Dichte der Stützflüssigkeit genau dem vorgegebenen Wert entsprechen, damit die Verdrängung der Stützflüssigkeit durch den Pfahlbeton auch erfolgt und es nicht zum Einschluß von Stützflüssigkeit in den Pfahlbeton kommt. Diese Einschlüsse stellen Fehlstellen dar, die die Tragkraft des Pfahles beeinträchtigen und mit erheblichem Aufwand saniert werden müssen.

Bei der Herstellung von Schlitzwänden wird der Boden lamellenweise mit Greifern ausgehoben und durch das Dichtungsmaterial ersetzt. Zur Stützung des offenen Schlitzes wird beim Zwei-Phasen-Verfahren eine Bentonit-Wasser-Suspension bzw. eine Bentonit-Wasser-Zement-Suspension beim Ein-Phasen-Verfahren verwendet. Die genaue Einhaltung der Suspensionsdichte spielt aus statischen Gründen eine wichtige Rolle. Der Standsicherheitsnachweis für den offenen Schlitz wird nach DIN 4126 geführt. Dabei geht neben den Eigenschaften des Baugrunds auch die Fliessgrenze der Stützsuspension ein, welche direkt von der Suspensionsdichte abhängt. Verändert sich die Suspensionsdichte, so verändern sich auch die übrigen Suspensionseigenschaften wie z.B. die Fließgrenze. Um den Erfolg der Baumassnahme und inbesondere die Stabilität des offenen Schlitzes zu gewährleisten, ist also eine kontinuierliche Kontrolle der Suspensionsdichte unabdingbar.

Es gibt nun nach dem Stand der Technik verschiedene Methoden, die Dichte von feinkörnigen Suspensionen zu messen. So kann die Dichte von Suspensionen z.B. mit Hilfe von radiometrischen Verfahren, die auf der Absorption von Strahlung aus einer radioaktiven Strahlungsquelle beruhen, gemessen werden. Die Suspension schwächt die auftreffende Strahlung, die Reststrahlung wird mit Hilfe eines Detektors aufgenommen und in ein Stromsignal umgewandelt. Dieses Verfahren liefert zwar sehr genaue Messwerte, erfordert aber für den Baustelleneinsatz die Beachtung der Strahlenschutzbestimmungen und ist darüberhinaus zeitaufwendig und kostspielig. Eine weitere Möglichkeit zur Dichtemessung besteht in der Verwendung eines Massedurchflussmessers. Das Messprinzip beruht auf der Ausnutzung der Coriolis-Kraft. Dabei wird ein mit Suspension durchströmtes Messrohr in eine Schwingung um seine Ruhelage versetzt. Die Suspensionsteilchen werden dadurch auf eine höhere Bahngeschwindigkeit beschleunigt, und ihre Masse erzeugt die Coriolis-Kraft, die der Beschleunigungsrichtung entgegenwirkt. Die Coriolis-Kraft erzeugt eine Verformung des Messrohres, die mit Sensoren erfasst werden kann. Auch dieses Verfahren ist ausreichend genau, kann aber unter Baustellenbedingungen nicht sinnvoll eingesetzt werden .

Das Verfahren, das üblicherweise nach dem Stand der Technik zur Dichtebestimmung feinkörniger Suspensionen auf der Baustelle benutzt wird, ist das Aräometerverfahren. Dieses Verfahren ist in der DIN 18 123 genormt. Dabei wird die Dichte der zu prüfenden Suspension durch Eintauchen einer Aräometerbirne in die Suspension gemessen. Je nach Dichteverteilung der Suspension schwimmt das Aräometer mehr oder weniger tief in der Suspension. Dieses Verfahren besitzt mehrere Nachteile. Zum einen ist es zeit- und personalintensiv. Es ist aus baustellenbetrieblichen Gründen nicht möglich, den Hersteilvorgang jedesmal zu unterbrechen, wenn eine Suspensionprobe untersucht wird und auf das Untersuchungsergebnis zu warten. Die Zeitspanne, die von der Feststellung eines Dichtewerts bis zur entsprechenden Korrektur der Suspensionsdichte in der Mischanlage vergeht, ist lang und gefährdet unter Umständen den Erfolg der Baumassnahme. Zum anderen liefert dieses Verfahren nur stichprobenartige Dichtewerte. Wünschenswert ist daher ein Verfahren, welches die Suspensionsdichte zu jedem beliebigen Zeitpunkt ohne zusätzlichen Aufwand an Personal und Zeit bestimmt.

Es ist daher Aufgabe der Erfindung, ein Verfahren und eine Vorrichtung zur kontinuierlichen Dichtemessung anzugeben, welches zum einen unter Baustellenbedingungen problemlos einsetzbar ist und welches die Dichte sowohl von frisch angemischten als auch von wiederaufbereiteten Suspensionen zu jedem Zeitpunkt ohne zusätzlichen Aufwand von Zeit und Personal angibt.

Das erfindungsgemäße Verfahren und die erfindungsgemässe Vorrichtung lösen die voranstehende Aufgabe durch die kennzeichnenden Merkmale der Patentansprüche 1 und 16. Danach wird die Suspensionsdichte in einem Suspensionsbehälter mit einem definierten Querschnitt durch Messung der Füllstandshöhe der Suspension und Wägung des Suspensionsgewichts kontinuierlich oder getaktet in Zeitabständen von 2 sek - 10 min ermittelt.

Erfindungsgemäss ist erkannt worden, dass es möglich ist, die Dichte einer feinkörnigen, im Tiefbau verwendeten Suspension zu jedem beliebigen Zeitpunkt und ohne zusätzlichen Zeit- und Personalaufwand zu messen, indem die Füllstandshöhe der Suspension in einem Suspensionsbehälter mit einem definierten Querschnitt und das Suspensionsgewicht gemessen und daraus kontinuierlich oder getaktet in Zeitabständen von 2 sek - 10 min die Suspensionsdichte ermittelt wird.

Zur Bestimmung der Dichte ρ einer Suspension ist die Kenntnis zweier weiterer physikalischer Grössen notwendig, nämlich des Volumens V und der Masse m. Die Dichte ρ errechnet sich dann aus dem Quotienten aus Volumen V und Masse m. Das Volumen der Suspension kann ermittelt werden, indem ein Behälter mit einem definierten und bekannten Querschnitt A verwendet wird, in dem die Füllhöhe H einer Suspension ermittelt wird. Das Volumen V ergibt sich dann aus dem Produkt des Querschnitts A und der Füllhöhe H. Die Masse der Suspension m wird durch Wägung des Suspensionsgewichts G ermittelt. Die Masse der Suspension m ergibt sich dann aus dem Quotient des Suspensionsgewichts G und der Erdbeschleunigung g. Nach dem erfindungsgemässen Verfahren kann nun zu jedem beliebigen Zeitpunkt kontinuierlich oder getaktet in Zeitabständen von 2 sek - 10 min die Suspensionsdichte ρ aus der Messung der Füllstandshöhe H und der gleichzeitigen Bestimmung des Suspensionsgewichtes G ermittelt werden.

Nach der Erfindung ist es also problemlos möglich, die Dichte sowohl von frisch angemischten, von wiederaufbereiteten oder von Mischungen zwischen frisch angemischten und wiederaufbereiteten Suspensionen zu bestimmen. Vorzugsweise wird die Dichte gemittelt aus 5 - 20 vorher einzeln ermittelten Meßwerten, um einzelne Meßwertausreißer zu den Dichten der verwendeten Suspension zu kompensieren. Das Gewicht des Suspensionsbehälters wird durch eine Nullmessung bestimmt.

In einer besonders vorteilhaften Ausgestaltung der Erfindung ist es möglich, zur Messung der Füllstandshöhe der Suspension einen oder mehrere Füllstandsmesseinrichtungen zu verwenden. Für diese Füllstandsmesseinrichtungen können z.B. Abtastgeräte, Schwimmerschalter, Druckschalter oder andere Füllstandsensoren und deren Kombinationen verwendet werden.

Die Wägung des Suspensionsgewichtes kann zweckmäßigerweise durch eine oder mehrere Wägeeinrichtungen erfolgen. Diese Wägeeinrichtungen können beispielsweise eine oder mehrere elektronische oder mechanische Waagen sein. Die ermittelten Daten werden in einem Prozessrechner verarbeitet.

Eine erfindungsgemäße Vorrichtung besteht aus einem Suspensionsbehälter mit einem definierten Querschnitt, einer Füllstandsmesseinrichtung, eine Wägeeinrichtung und einem Prozessrechner. Der Querschnitt des Suspensionsbehälters kann beliebige Formen annehmen, besonders vorteilhaft ist ein Querschnitt in rechteckiger, runder oder ovaler Form.

Es gibt nun verschiedene Möglichkeiten, die Lehre der vorliegenden Erfindung in vorteilhafter Weise auszugestalten und weiterzubilden. Dazu wird einerseits auf die dem Patentanspruch 1 nachgeordneten Ansprüche, andererseits auf die nachfolgende Erläuterung von drei Ausführungsbeispielen der Erfindung verwiesen. In Verbindung mit der Erläuterung der bevorzugten Ausführungsbeispiele der Erfindung werden auch im allgemeinen bevorzugte Ausgestaltungen und Weiterbildungen der Lehre erläutert.

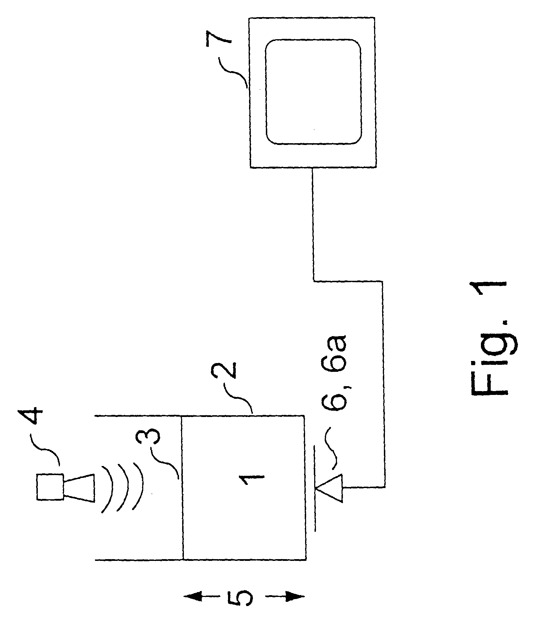

In Fig. 1 wird zunächst der prinzipielle Aufbau einer Anlage für das erfindungsgemässe Verfahren erläutert. Die Suspension 1 befindet sich in einem Suspensionsmischbehälter 2 und bildet einen Suspensionsspiegel 3. Die Füllstandshöhe 5 der Suspension 1 wird mit Hilfe eines Füllstandssensors 4 bestimmt. Das Gewicht der Suspension 1 wird durch eine elektronische Waage 6 oder eine mechanische Waage 6a bestimmt. Die ermittelten Daten werden mit einem Prozessrechner 7 verarbeitet.

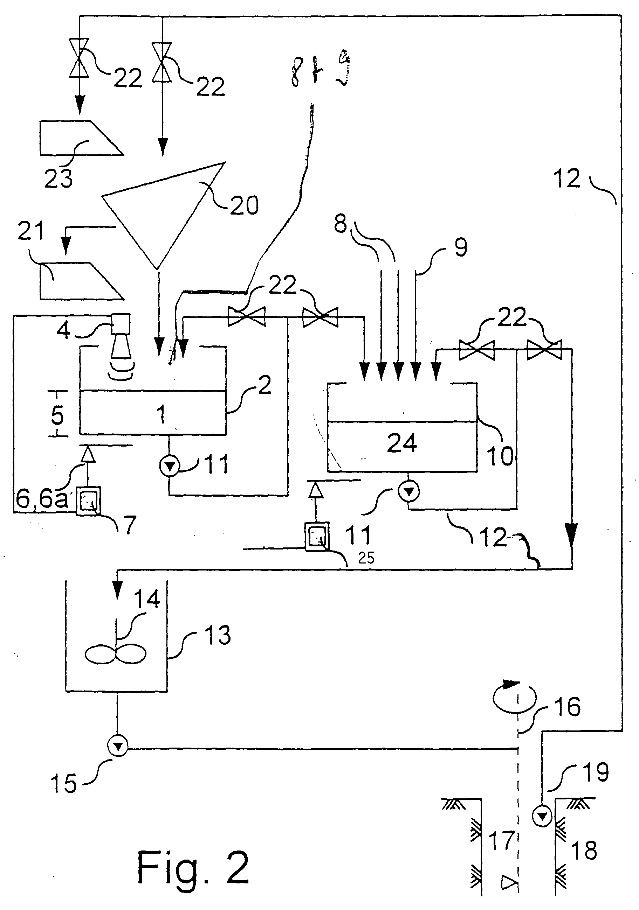

Fig. 2 zeigt das Verfahrensschema für die Anwendung des erfindungsgemässen Verfahrens für den Rückfluß von Suspension 1 am Beispiel einer Baumassnahme, bei der das Düsenstrahlverfahren verwendet wird. Zuschlagsstoffe 8 und Wasser 9 werden zunächst im gewünschten Verhältnis in einem Suspensionsmischer 10 angemischt und mit Hilfe einer Austragspumpe 11 über Rohrleitungen 12 in einen Vorratsbehälter 13 gepumpt. Dort wird die Suspension 1 durch einen Mischflügel 14 ständig in Bewegung gehalten und gerührt. Die Suspension 1 wird über eine Hochdruckpumpe 15 aus dem Vorratsbehälter 13 in das Bohrgestänge 16 gepumpt und mischt sich im Bohrloch 17 mit dem umgebenden Bodenmaterial 18. Der Rückfluß an gebrauchter Suspension, die mit Bodenmaterial 18 aufgeladen ist, kann entweder mittels einer Bohrlochpumpe 19 durch eine weitere Rohrleitung 12 in eine Regenerieranlage 20 gepumpt werden oder, falls erforderlich, in einem Deponierbehälter für nicht regenerierbare Suspension 23 gesammelt werden. Die Regelung des Suspensionsflusses erfolgt durch Absperrventile 22, die je nach Erfordernis die verschiedenen Fließwege absperren können. Das abgetrennte Bodenmaterial wird in einem Deponierbehälter 21 gesammelt und entsorgt. Die wieder aufbereitete Suspension wird in den Suspensionsmischbehälter 2 gegeben, wo ihre Füllstandshöhe 5 mit einem Füllstandssensor 4 bestimmt und gleichzeitig ihr Suspensionsgewicht durch eine elektronische Waage 6 oder eine mechanische Waage 6a ermittelt wird. Dabei ist der Prozessrechner 7 mit dem Füllstandssensor 4 und der Wägeeinrichtung verbunden. Entspricht die Dichte der Suspension nicht den Vorgaben, können Zuschlagsstoffe 8 und Wasser 9 entsprechend den Erfordernissen zu dosiert werden, damit eine konstante Dichte erzielt und gehalten werden kann. Eine Pumpe 11 sorgt für eine Homogenisierung der Mischung 1 in dem der Suspensionsmischbehälter 2 verwirbelt oder kontinuierlich umgeschichtet wird. Eine konstante Menge der Mischung 1 wird in Behälter 10 mit den Zuschlagstoffen 8 und 9 zur aufbereiteten Suspension vermengt. Die so aufbereitete Suspension fliesst dann über den Vorratsbehälter 13 zur Hochdruckpumpe 15 zurück.

Die Ergebnisse des Prozessrechners 7 können alternativ an einen Wiegeprozessor 25 übermittelt werden, der die erforderlichen Mengen der Zuschlagstoffe 8 und 9 zur herstellung einer konstant gleichmäßigen Mischung in Behälter 10 ermittelt.

Alle Zugaben können kontinuierlich oder chargenweise aufgegeben werden.

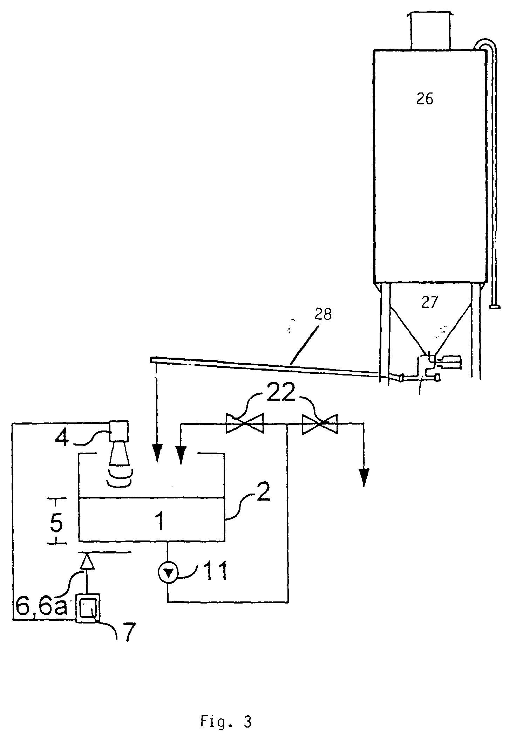

Fig. 3: Von einem Baustellensilo 26 gelangen Trockenbaustoffe über einen Auslaßtrichter 27 in eine Austragvorrichtung 28. Die Austragvorrichtung 28 kann mit Schneckenförderern und Mischern (nicht dargestellt) ausgestattet sein. In die Austragvorrichtung 28 mündet eine Wasserzufuhr. Das Bereitstellungsgemisch aus Trockenbaustoffen und Wasser wird dem Suspensionsmischbehälter 2 aufgegeben, wo die Füllstandshöhe 5 mit einem Füllstandssensor 4 bestimmt und gleichzeitig das Gewicht des Bereitstellunggemisches durch eine elektronische Waage 6 oder eine mechanische Waage 6a ermittelt wird. Dabei ist der Prozessrechner 7 mit dem Füllstandssensor 4 und der Wägeeinrichtung verbunden. Entspricht die Dichte des Bereitstellunggemisches nicht den Vorgaben, können entweder die Zugabe von Trockenbaustoffen durch Öffnen oder Schließen einer Klappe oder durch Regelung der Drehzahl einer Zellradschleuse, Schnecke oder ähnlichem (nicht dargestellt) zwischen Baustellensilo 26 und Auslaßtrichter 27 oder die Zugabe von Flüssigkeit wie Wasser oder einer Bentonit-Suspension in die Austragvorrichtung 28 so variiert werden, bis die Vorgaben erfüllt sind. Zu diesem Zweck ist der Prozessrechner 7 mit Stellgliedern (nicht dargestellt) an der Zellradschleuse oder an der Wasserzufuhr in die Austragvorrichtung 28 verbunden.

Das Bereitstellunggemisch aus dem Suspensionsbehälter 2 ist zur Weiterverarbeitung fertig.

Gemäß einer vorteilhaften Ausgestaltung sind Prozessrechner 7 und Wiegeprozessor 25 der oben aufgeführten Ausführungsbeispiele mit Einrichtungen zur Erfassung der Meßwerte in gleichmäßigen Zeitabständen von 2 sek bis 10 min ausgestattet. Die Ermittlung der Suspensionsdichte kann vorzugsweise aus 5 - 20 Meßwerten gemittelt werden, so daß die Vorrichtung geringere Empfindlichkeit gegen "Meßwertausreißer" aufweist.

### Bezugszeichenliste:

- 1: Suspension
- 2: Suspensionsmischbehälter
- 3: Suspensionsspiegel
- 4: Füllstandssensor
- 5: Füllstandshöhe
- 6: elektronische Waage
- 6a: mechanische Waage
- 7: Prozessrechner
- 8: Zuschlagsstoffe
- 9: Wasser
- 10: Suspensionsmischer
- 11: Austragspumpe
- 12: Rohrleitungen
- 13: Vorratsbehälter
- 14: Mischflügel
- 15: Hochdruckpumpe
- 16: Bohrgestänge
- 17: Bohrloch
- 18: Bodenmaterial
- 19: Bohrlochpumpe
- 20: Regenerieranlage
- 21: Deponierbehälter für separierte Feststoffe
- 22: Absperrventil
- 23: Deponierbehälter für nicht regenerierte Suspension
- 24: Verarbeitungsfertige Suspension
- 25: Wiegeprozessor
- 26: Baustellensilo
- 27: Auslaßtrichter
- 28: Austragvorrichtung

## Patentansprüche

1. Verfahren zur Ermittlung der Dichte feinkörniger, im Tiefbau verwendeter Suspensionen, wie insbesondere Bentonit-Wasser-Suspensionen, Bentonit-Zement-Wasser-Suspensionen, Bentonit-Wasser-Zement-Sand-Suspensionen und dergleichen, **dadurch gekennzeichnet**, **daß** die Suspensionsdichte in einem Suspensionsbehälter (2) mit einem definierten Querschnitt durch Messung der Füllstandshöhe (5) der Suspension (1) und Wägung des Suspensionsgewichts kontinuierlich oder getaktet in Zeitabständen von 2 sek - 10 min ermittelt wird.

2. Verfahren nach Anspruch 1, dadurch gekennzeichnet, daß es sich bei der Suspension (1) um eine frisch angemischte Suspension handelt.

3. Verfahren nach einem der Ansprüche 1 bis 2, dadurch gekennzeichnet, daß es sich bei der Suspension (1) um eine aufbereitete Suspension handelt.

4. Verfahren nach einem der Ansprüche 1 bis 3, dadurch gekennzeichnet, daß die Ermittlung der Supensionsdichte alle 5 - 20 Meßwerte gemittelt durchgeführt wird.

5. Verfahren nach einem der Ansprüche 1 bis 4, dadurch gekennzeichnet, daß das Gewicht des Suspensionsbehälters durch eine Nullmessung ermittelt wird.

6. Verfahren nach einem der Ansprüche 1 bis 5, dadurch gekennzeichnet, daß die Messung der Füllstandshöhe (5) der Suspension (1) durch einen oder mehrere Füllstandsmesseinrichtungen erfolgt.

7. Verfahren nach einem der Ansprüche 1 bis 6, dadurch gekennzeichnet, daß die Messung der Füllstandshöhe (5) der Suspension (1) durch ein oder mehrere Abtastgeräte erfolgt.

8. Verfahren nach einem der Ansprüche 1 bis 6 , dadurch gekennzeichnet, daß die Messung der Füllstandshöhe (5) der Suspension (1) im Suspensionsbehälter (2) durch einen oder mehrere Schwimmerschalter erfolgt.

9. Verfahren nach einem der Ansprüche 1 bis 6, dadurch gekennzeichnet, daß die Messung der Füllstandshöhe (5) der Suspension (1) im Suspensionsbehälter (2) durch einen oder mehrere Druckschalter erfolgt.

10. Verfahren nach einem der Ansprüche 1 bis6 , daß die Messung der Füllstandshöhe (5) der Suspension (1) im Suspensionsbehälter (2) durch einen oder mehrere Füllstandssensoren erfolgt.

11. Verfahren nach einem der Ansprüche 1 bis 10, dadurch gekennzeichnet, daß die Messung der Füllstandshöhe (5) der Suspension (1) im Suspensionsbehälter (2) durch eine Kombination der Füllstandsmesseinrichtungen der Ansprüche 7 bis 10 erfolgt.

12. Verfahren nach einem der Ansprüche 1 bis 11, dadurch gekennzeichnet, daß die Wägung des Suspensionsgewichtes durch eine oder mehrere Wägeeinrichtungen erfolgt.

13. Verfahren nach einem der Ansprüche 1 bis 12, dadurch gekennzeichnet, daß die Wägung des Suspensionsgewichtes durch eine oder mehrere elektronische Waagen (6) erfolgt.

14. Verfahren nach einem der Ansprüche 1 bis 13, dadurch gekennzeichnet, daß die Wägung des Suspensionsgewichtes durch eine oder mehrere mechanische Waagen (6a) erfolgt.

15. Verfahren nach einem der Ansprüche 1 bis 14, dadurch gekennzeichnet, daß die ermittelten Daten in einem Prozessrechner (7) verarbeitet werden.

16. Vorrichtung zur Durchführung des Verfahrens zur Ermittlung der Dichte feinkörniger, im Tiefbau verwendeter Suspensionen, wie insbesondere Bentonit-Wasser-Suspensionen, Bentonit-Zement-Wasser-Suspensionen, Bentonit-Wasser-Zement-Sand-Suspensionen und dergleichen nach den Ansprüchen 1 bis 15,
**dadurch gekennzeichnet**, **daß** sie aus einem Suspensionsbehälter (2) mit einem definierten Querschnitt, einer Füllstandsmesseinrichtung, einer Wägeeinrichtung und einem Prozessrechner (7) besteht.

17. Vorrichtung nach Anspruch 16, dadurch gekennzeichnet, daß der oder die Füllstandsmesseinrichtungen Ultraschallsensoren sind.

18. Vorrichtung nach Anspruch 16, dadurch gekennzeichnet, daß die Füllstandsmesseinrichtungen Abtastgeräte sind.

19. Vorrichtung nach Anspruch 16, dadurch gekennzeichnet, daß die Füllstandsmesseinrichtungen Schwimmerschalter sind.

20. Vorrichtung nach Anspruch 16, dadurch gekennzeichnet, daß die Füllstandsmesseinrichtungen Druckschalter sind.

21. Vorrichtung nach einem der Ansprüche 16 bis 20, dadurch gekennzeichnet, daß der Querschnitt des Suspensionsbehälters (2) eine rechteckige Form aufweist.

22. Vorrichtung nach einem der Ansprüche 16 bis 21, dadurch gekennzeichnet, daß der Querschnitt des Suspensionsbehälters (2) eine runde Form aufweist.

23. Vorrichtung nach einem der Ansprüche 16 bis 22, dadurch gekennzeichnet, daß der Querschnitt des Suspensionsbehälter (2) eine ovale Form aufweist.

24. Vorrichtung nach einem der Ansprüche 16 bis 22, dadurch gekennzeichnet, daß von einem Baustellensilo (26) Trockenbaustoffe über einen Auslaßtrichter (27) in eine Austragvorrichtung (28) gelangen und in die Austragvorrichtung (28) eine Wasserzufuhr mündet zur Bereitstellung eines Gemischs aus Trockenbaustoffen und Flüssigkeiten, die dem Suspensionsmischbehälter (2) aufgegeben wird.

25. Vorrichtung nach einem der Ansprüche 16 bis 22, dadurch gekennzeichnet, daß die Austragvorrichtung (28) mit Schneckenförderern und Mischern ausgestattet ist.
